# EUROPEAN PATENT SPECIFICATION

(11) **EP 1 909 773 B1**
(45) Date of publication and mention of the grant of the patent: **14.09.2011**
(21) Application number: 06779913.0
(22) Date of filing: 13.07.2006
(51) Int. Cl.: A61K 9/70, A61K 9/12, A61K 31/4995

(54) **TRANSDERMAL SYSTEM FOR VARENICLINE**
TRANSDERMALES SYSTEM FÜR VARENICLIN
SYSTÈME TRANSDERMIQUE POUR LA VARENICLINE

(30) Priority: 26.07.2005 US 702961 P
(43) Date of publication of application: 16.04.2008
(73) Proprietor: Pfizer Products Inc., Groton, CT 06340 (US)
(72) Inventor: JOHNSON, Barbara, Alice, Groton, CT 06340 (US); ZIEGLER, Carl, Bernard, Groton, CT 06340 (US)
(74) Representative: Hodge, Emma Jane
(86) International application number: PCT/IB2006/002081
(87) International publication number: WO 2007/012963

(56) References cited:
- WO-A-02/092089
- WO-A-03/045394
- WO-A-2006/040680
- WO-A-2006/072832
- WO-A2-03/005998

## Description

### 1. Field of the Invention

The present invention relates to pharmaceutical compositions for medicinal uses thereof.

### 2. Background Art

Varenicline has the structure: 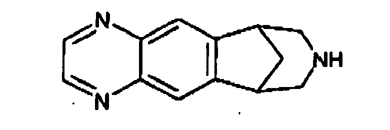 Varenicline is also known as 5,8,14-triazatetracyclo[10.3.1.0^{2,11}.0^{4,6}]-hexadeca-2(11)3,5,7,9-pentaene or 7,8,9,10-tetrahydro-6,10-methano-6H-pyrazino[2,3-h][3]-benzazepine. Varenicline and pharmaceutically acceptable acid addition salts thereof are referred to in International Patent Publication WO 99/35131, published July 15, 1999.
International Patent Publication WO 03/005998 concerns pharmaceutical compositions and methods of modulating cholinergic function in a mammal comprising administering nicotinic receptor partial agonists or pharmaceutically acceptable salt thereof; and an anti-ematic/anti-nausea agent or pharmaceutically acceptable salt thereof and a pharmaceutically acceptable carrier.
International Patent Publication WO 03/045394 concerns succinic acid salts of 5,8,14-triazatetracyclo[10.3.1.0(2,11).0(4,9)-hexadeca-2(11),3,5,7,9-pentaene and pharmaceutical compositions thereof.
International Patent Publication WO 02/092089 concerns tartrate salts of 5,8,14-triazatetracyclo[10.3.1.0(2,11).0(4.9)-hexadeca-2(11),3,5,7,9-pentaene and pharmaceutical compositions thereof.

Varenicline binds to neuronal nicotinic acetylcholine specific receptor sites and is useful in modulating cholinergic function. Accordingly, this compound Is useful in the treatment of various conditions or diseases including, but not limited to, inflammatory bowel disease (including, but not limited to, ulcerative colitis, pyoderma gangrenosum and Crohn's disease), irritable bowel syndrome, spastic dystonia, chronic pain, acute pain, celiac sprue, pouchitis, vasoconstriction, anxiety, panic disorder, depression, bipolar disorder, autism, sleep disorders, jet lag, amyotrophic lateral sclerosis (ALS), cognitive dysfunction, hypertension, bulimia, anorexia, obesity, cardiac arrhythmias, gastric acid hypersecretion, ulcers, pheochromocytoma, progressive supranuclear palsy, chemical dependencies and addictions (e.g., dependencies on, or addictions to nicotine (and/or tobacco products), alcohol, benzodiazepines, barbiturates, opioids or cocaine), headache, migraine, stroke, traumatic brain injury (TBI), obsessive-compulsive disorder (OCD), psychosis, Huntington's chorea, tardive dyskinesia, hyperkinesia, dyslexia, schizophrenia, multi-infarct dementia, age-related cognitive decline, epilepsy, including petit mal absence epilepsy, senile damentia of the Alzheimer's type (AD), Parkinson's disease (PD), attention deficit hyperactivity disorder (ADHD) and Tourette's Syndrome.

Varenicline is a highly potent compound such that dosage forms are necessarily highly diluted with excipients. The excipients provide dosage forms with adequate stability, while also providing for such desirable features as controlling the drug dissolution (e.g., either fast dissolving or slow dissolving in a controlled-release system as described in co-pending applications U.S. Patent Publication No. 2003-0180360 A1, published Sept. 25, 2003, and Serial No. 10/848,464, flied May 18, 2004), masking bad taste, and providing appropriate properties for preparation of the dosage form (i.e., compression properties for tablets). Finally, because of the high dilution with excipients, reactivity of varenicline with the excipients themselves or with trace impurities (i.e., degradants) of the excipients can be especially problematic.

There are advantages of delivering varenicline in the form of a transdermal composition. For example, relative to an oral dosage form such as a tablet or capsule delivery of varenicline via a transdermal composition would be a preferred choice by patients who have difficulty in swallowing tablets, capsules or other solids.

The tablet dosage form of varenicline has shown, In some instances, a certain level of nausea in patients. There is a need to reduce these side effects. A gradual release of the varenicline dosage form such as would be the case from a transdermal composition might prove to be useful towards reducing the incidence of nausea and enhance the desirability of the drug to a larger patient population requiring its use. Finally, it is likely to assume there would be a higher compliance rate if a patient could apply a transdermal patch that delivers therapeutically useful levels of active ingredient over the course of a day or a longer period of time versus taking a once or twice daily tablet or capsule with water. Accordingly, there is a need for providing transdermal dosage forms of varenicline.

### SUMMARY OF THE INVENTION

The present invention relates to the transdermal application of varenicline and its pharmaceutically acceptable salt forms, herein referred to as the active ingredient and methods for delivering it to an individual, in particular to such compositions in adhesive coated sheet form that are suitable for administration to skin to provide a local or systemic therapeutic effect. The composition comprises a backing sheet that is flexibly conformable to the area of skin, the backing sheet having opposing surfaces that are respectively distal and proximal to the skin when applied; and a coating on the proximal surface of the backing sheet. The coating comprises (a) an adhesive and (b) a water-soluble active agent comprising varenicline or salt thereof, the active agent being in a therapeutically effective total amount and dispersed in a matrix that comprises zero to less than an active agent solubilizing effective amount in total of one or more solvents other than the adhesive.

When one or more solvents other than the adhesive, for example water or a polyhydric alcohol such as polyethylene glycol or propylene glycol, are defined herein as present in the matrix at "less than an active agent solubilizing effective amount in total", it will be understood that the amount of such solvents is insufficient to dissolve all of the active agent present in the coating. The active agent can be dispersed in solid particulate form in the matrix. Alternatively, where the matrix is formed predominantly by the adhesive, the active agent can optionally be wholly or partly molecularly dispersed, i.e., in solid solution, in such a matrix,

The invention also relates to processes for preparing such compositions and to methods of treatment comprising administration of such compositions to skin of a subject in need thereof.

The present compositions are not limited by any process used to prepare then. In an illustrative process, the active agent is dissolved in a solvent or mixture of solvents, for example ethanol and water, prior to mixing with the adhesive. However, typically the solvent or mixture of solvents is later removed by heating, thus the final composition, in accordance with the present invention, contains no such solvents or has less than an active agent solubilizing effective amount in total of such solvents.

In a preferred embodiment the coating comprises a layer having the active agent dispersed in a matrix that comprises the adhesive. Alternatively, the coating can comprise two layers: a reservoir layer that comprises the active agent adjacent to the backing sheet, and an adhesive layer that is proximal to the skin when applied. Optionally in such a coating, a membrane that permits passage of the active agent is present between then reservoir layer and the adhesive layer.

In preferred compositions, the coating further comprises one or more skin permeation enhancers- Preferably a peelable release liner is also provided. This liner, prior to use, is adjacent to the layer that contains the adhesive, and is removed prior to application of the composition to the skin.

Another embodiment is a method of making a pressure sensitive adhesive matrix patch for transdermal delivery using the active ingredient. In a specific embodiment for transdermal administration, the active ingredient can be contained in a matrix from which it is released in the desired gradual, constant and controlled manner. The permeability of the matrix during the release of the active ingredient is based on diffusion. There is further provided a method of systemic treatment of a subject, the method comprising applying a pharmaceutical composition as provided herein to a skin surface of the subject, and leaving the composition in place for a time period effective to permit transdermal delivery of a therapeutic amount of the active agent.

### DETAILED DESCRIPTION OF THE INVENTION

The present invention relates to the transdermal application of varenicline and its pharmaceutically acceptable salt forms, herein referred to as the active ingredient and methods for delivering it to an individual, in particular to such compositions In adhesive coated sheet form that are suitable for administration to skin to provide a local or systemic therapeutic effect. The present invention utilizes varenicline or its pharmaceutically acceptable salt as the active ingredient. Varenicline can be used per se or in the form of its pharmaceutically acceptable salt, solvate and/or hydrate. Although any pharmaceutically acceptable form of varenicline can be used in connection with the present invention, it is preferable to use a salt form of the drug. A particularly preferred salt form of the drug is the L-tartrate salt.

In particular, the present Invention provides for the transdermal composition of the Invention for use in reducing nicotine addiction or aiding in the cessation or lessening of tobacco use in a subject, wherein an amount of the varenicline that is effective in reducing nicotine addiction or aiding in the cessation or lessening of tobacco use is administered to a subject via administration of a transdermal dosage form of the drug.

The present invention can be used to treat disorders or conditions including, but not limited to, inflammatory bowel disease, ulcerative colitis, pyoderma gangrenosum, Crohn's disease, Irritable bowel syndrome, spastic dystonia, chronic pain, acute pain, celiac sprue, pouchitis, vasoconstriction, anxiety, panic disorder, depression, bipolar disorder, autism, sleep disorders, jet lag, amyotrophic lateral sclerosis (ALS), cognitive dysfunction, hypertension, bulimia, anorexia, obesity, cardiac arrhythmias, gastric acid hypersecretion, ulcers, pheochromocytoma, progressive supranuclear palsy, chemical dependencies and addictions; dependencies on, or addictions to, nicotine, tobacco products, alcohol, benzodiazepines, barbiturates, opioids or cocaine; headache, stroke, traumatic brain injury (TBI), obsessive-compulsive disorder (OCD), psychosis. Huntington's Chorea, tardive dyskinesia, hyperkinesia, dyslexia, schizophrenia, multi-infarct dementia, age related cognitive decline, epilepsy, petit mal absence epilepsy, senile dementia of the Alzheimer's type (AD), Parkinson's disease (PD), attention deficit hyperactivity disorder (ADHD), Tourette's Syndrome; and any other similar disorder or condition known to those of skill in the art.

The term "varenicline," as used herein means the drug that binds to neurona nicotinic acetylcholine specific receptor sites, and is useful in modulating cholinergic function. Varenicline has the general formula of: 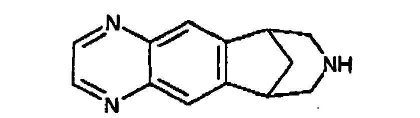 Varenicline includes the parent drug and all pharmaceutically acceptable salts thereof. The parent drug of varenicline is described in International Patent Publication WO 99/35131, published July 15, 1999. In any of the embodiments, varenicline or any of its pharmaceutically acceptable salts, solvates and/or hydrates can be used. Procedures for making varenicline are described in U.S. Patent No. 6,410,550. The resolution of racemic mixtures of varenicline is described In WO01/62736.

The term "mgA" refers to the number of milligrams of active drug based on the free base form of the drug.

The term "pharmaceutically acceptable" means the substance or composition must be compatible chemically, physically, - and/or toxicologically, with the other components comprising a formulation, and/or the mammal being treated therewith.

The term "pharmaceutically acceptable salt" means non-toxic acid addition salts derived from inorganic and organic acids. Suitable salt derivatives include, but are not limited to, halides, thiocyanates, sulfates, bisulfates, sulfites, bisulfites, arylsulfonates, alkylsulfates, phosphonates, monohydrogen-phosphates, dihydrogenphosphates, metaphosphates, pyrophosphonates, alkanoates, cycloalkylalkanoates, arylalkonates, adipates, alginates, aspirates, benzoates, fumarates, glucoheptanoates, glycerophosphates, lactates, maleates, nicotinates, oxalates, palmitates, pectinates, picrates, pivalates, succinates, tartarates, citrates, camphorates, camphorsulfonates, digluconates, trifluoroacetates, and the like,

The term "active ingredient" means the therapeutically active compound, varenicline, as well as any prodrugs thereof and pharmaceutically acceptable salts, hydrates, and solvates of the compound and the prodrugs.

The term " ingredients" means any excipients, diluents, solvents, permeation enhancer, preservatives, buffers, gel forming agents, lubricants, carriers, stabilizers, gels, dyes, pigments, surfactants, inert fillers, tackifies, texturizers, softeners, emulsifiers, and mixtures thereof that are formulated with varenicline or any pharmaceutically acceptable salts, hydrates, and solvates of this drug.

The term "appropriate period of time" or "suitable period of time" means the period of time necessary to achieve a desired effect or result. For example, a mixture can be blended until a potency distribution is reached that is within an acceptable range for a given application or use of the blended mixture.

The term "unit dose," "unit dosage." or "unit dosage form" means a physically discrete unit that contains a predetermined quantity of active ingredient calculated to produce a desired therapeutic effect.

The term "effective amount as used herein means the amount determined by such considerations as are known in the art of reducing nicotine addiction or aiding in the cessation or lessening of tobacco use in an individual, wherein it must be effective to provide measurable relief in treated individuals such as exhibiting improvements including but not limited to, more rapid recovery, improvement or elimination of symptoms or reduction of complications, lack of dependency upon nicotine-containing compounds, lack of desire towards nicotine-containing compounds, or other measurements as appropriate and known to those skilled in the medical arts.
The present invention has numerous embodiments. In any of the embodiments, pharmaceutical compositions of varenicline can be desirably administered in doses ranging from about 0.1 mgA up to about 6 mgA per day (where mgA refers to mg of active drug based on the free base form of the drug), more preferably from about 0.5 to 4 mgA/day, and most preferably from about 1 to 4 mgA per day in single or divided doses. Variations in such dosages, however, necessarily occur depending upon the weight and condition of the subject being treated. Depending on individual responses, dosage levels below the lower limit of the aforesaid range can be more than adequate, while in other cases still larger doses can be employed without causing any harmful side effects. The final pharmaceutical composition is processed into a unit dosage form and then packaged for distribution. The processing step varies depending upon the particular unit transdermal dosage form. Those of skill in the art are well aware of the procedures used for manufacturing the various unit dosage forms.

The present invention provides transdermal compositions containing the active ingredient and methods for delivering it to an individual.The active ingredient in the transdermal composition is gradually released into the systemic circulation through the skin. Moreover, it is reasonable to assume that the active Ingredient within transdermal composition, is released through the skin over a suitable period of time. It is believed that the controlled release of the active ingredient from the transdermal composition prevents excessive concentrations of the drug in the body which in turn reduces or prevents the nausea side effect.

A pharmaceutical composition of the invention is described herein as an "adhesive coated sheet", a generic term which will be understood to embrace patches, tapes, poultices, pads, plasters, cataplasms and dressings that are adhesive to skin. The components of the adhesive coated sheet are described herein with reference to a skin surface to which the composition Is to be applied. As applied to a layer or surface herein, the term "proximal" means toward the skin surface and the term "distal" means away from the skin surface, when the composition is correctly applied.

The most distal layer of the composition is a backing sheet that is flexibly conformable to the skin surface. Any suitable material can be used for the backing sheet, but typically a polymer film, e.g., one comprising one or more of polyethylene, polyvinyl chloride ethyl vinyl acetate, polyurethane and polyester, or a woven or nonwoven fabric, optionally having a polymer film laminated thereon, is used. The backing sheet can be airtight and/or waterproof, providing a substantially occlusive dressing. Alternatively, a backing sheet can be used having pores or other means for circulation of air to the treated skin, area. A presently preferred backing sheet is an ethyl vinyl acetate film having a thickness of about 20 to about 100 µ*m,* for example Modified 1200 of Mylan Technologies, Inc.
Transdermal delivery can be achieved by several means. Medicinal bandage type delivery devices use a membrane-controlled system or a matrix system in which the active ingredient reservoir is in the adhesive layer (S. Venkatraman, N. Davar, A. Chester, and L. Kleiner in "An Overview of Controlled Release Systems" pp. 445-452 in Handbook of Pharmaceutical Controller Release Technology ed Donald L. Wise, Marcel Dekker, Inc., NY 2000.) Alternate means include spraying on the active ingredient from a liquid such that due to evaporation of the liquid the active ingredient is in a more concentrated form on the skin. This type of embodiment is exemplified by U.S. 5,958,379. The active ingredient could also be applied to the skin in the form of an ointment, cream, lotion, solution or plaster or by other means known by those experienced in the art.

In a specific embodiment for transdermal administration, the active ingredient can be contained in a matrix from which it is released in the desired gradual, constant and controlled manner, The permeability of the matrix during the release of the active ingredient is based on diffusion. A system of this type is described in the U.S. Patent No. 4,769,028. It consists of an impermeable backing layer, an active compound reservoir associated with and made of a polymer compound, a contact adhesive layer that is permeable to the active ingredient, associated with the reservoir and a protective layer covering the contact-adhesive layer, which can be removed for use. Other embodiments include reservoir layers that have a high intrinsic tackiness that can be used simultaneously as the contact-adhesive layer.

One useful embodiment is a method of making a pressure sensitive adhesive matrix patch for transdermal delivery using hydrophilic active ingredients and their sails as described in US 6,365,178 B1. The steps of making one embodiment of the matrix transdermal device are:
a. dissolving an effective amount of hydrophilic active ingredient with an aqueous dispersion, comprising a water phase, of a hydrophobic pressure sensitive adhesive and optionally a permeation enhancer, to form a mixture;
b. film casting the mixture and evaporating the water phase to obtain a hydrophobic pressure sensitive adhesive matrix film having the active ingredient fully dissolved therein and having first and second surfaces, thereof; and
c. laminating a release liner to the first surface of the matrix film and and a substantially drug-impermeable backing layer to the second surface.

Preferably the water-based adhesive, is an acrylic copolymer, ethylene-vinyl acetate copolymer, or polyisobutylene polymer or copolymer that is a two-phase dispersion of water-insoluble polymer particles in water. The pressure sensitive adhesives must also be pharmaceutically acceptable.
The backing layer, which adheres to the active ingredient containing adhesive layer serves as the upper layer of the patch during use and functions as the primary structural element of the transdermal device. The backing layer is made of a sheet of film of a preferably flexible elastomeric material that is substantially impermeable to the active ingredient and any permeation enhancer that may be present. The backing layer is typically about 0.001-0.004 inch in thickness and is preferably of a material that permits the transdermal device to be worn comfortably on any skin area. Examples of polymers useful for the backing layer may include, but are not limited to, polyethylene, polypropylene, polyesters, polyurathanes, polyethylene vinyl acetate, polyvinylidene chloride, block copolymers such as PEBAX, and the like. The backing layer can also comprise laminates of one or more of the foregoing polymers.

The release liner is a disposable element that serves only to protect the transdermal device prior to application to the skin. Typically the release liner is formed from a material impermeable to the active ingredient, permeation enhancer and any other components of the transdermal device, and is easily strippable form the pressure sensitive adhesive. Release liners can generally be made of the same materials as the backing layer.
The active ingredient adhesive matrix layer can, in addition to the adhesive, active ingredient and optional permeation enhancer, also contain other optional ingredients, such as thickeners, excipients, emulsifiers, tackifiers, preservatives, defoamers, antioxidants, surfactants and the like, which are nontoxic and do not Interfere with delivery of the active ingredient.

Other embodiments of the medicinal bandage include a rate controlling membrane and/or the active ingredient reservoir dispersed in a solid carrier, semi-solid carrier or ointment and are exemplified by US 4,752,478. The rate-controlling membrane can be a microporous membrane and can be made of any porous material allowing the passage of the active ingredient such as but not limited to micorporous polypropylene, microporous nylon, microporous polycarbonate. Other materials that can be used as rate-controlling membrane include ethylene vinyl acetate copolymers and polyethylene as disclosed In US 4,589,580 and other materials as disclosed in US 3.797,494.

Another embodiment of the rate-controlling membrane is exemplified by US 6,375,978 B1. In this embodiment the rate-controlling membrane may be pretreated by an annealing process to provide consistent membrane functionality over time and elevated storage conditions.

For a particular membrane material, the rate control of the transdermal drug delivery device is dependent on but not limited to the composition, pore size, and thickness of the rate-controlling membrane, the viscosity of the active ingredient formulation and any diffusive medium that can impregnate the pores.

The transdermal device may be comprised of an active ingredient reservoir layer between a backing layer and a contact adhesive layer, wherein the rate controlling membrane Is on the skin-proximal side of the active ingredient reservoir layer. The drug reservoir may optionally include permeation enhancers and/or other excipients. An alternate embodiment of this device has a separate permeation enhancer reservoir which is located on the skin proximal side of the backing layer and separated from the active ingredient reservoir which is proximal to the skin by the rate-controlling membrane. The membrane material may be selected from but not limited to the following materials: ethylene vinyl acetate copolymers, polyethylene, copolymers of ethylene, polyolefins including ethylene oxide copolymer such as Engage® (DuPont Dow Elastomers), polyamides, cellulosic materials, polyurethanes, polyether blocked amides copolymers such as PEBAX® (Elf Atochem North America, Inc.) and polyvinyl acetate.

The active ingredient and/or permeation enhancer reservoir(s) can be a gel or a polymer and may comprise an aqueous or non-aqueous composition. Suitable materials used in the reservoir include, but are not limited to gelled mineral oil, polyisobutylene, aluminum stearate, propylene glycol, fatty acid esters, natural and synthetic rubbers or other polymeric material, silicone fluids, polysiloxanes, polyacrylates, ethylene vinyl acetate copolymers, hydroxyethylene cellulose, hydroxypropyl cellulose, hydroxypropylmethylcellulose and petroleum jelly. Permeation enhancers include, but are not limited to ethanol and other higher alcohols, N-decylmethylsufoxide (nDMS), polyethylene glycol monolaurate, dilaurate and related esters, glycerol mono-oleate and related mono, di and trifunctional glycerides, diethyl toluamide, Azone® (a product of Nelson Research Corp.,) N,N-dimethyl lauramide, N,N-dimethyl lauramine oxide.

In addition to the active ingredient(s) and permeation enhancer(s) the transdermal device may include stabilizers, dyes, pigments, inert fillers, tackifiers, excipients and other components of transdermal delivery devices as are known in the art.

In one particular embodiment the rate controlling membrane is subjected to an annealing process to maintain a permeability within a preferred range even after the transdermal device is exposed to elevated temperatures. A preferred embodiment uses a rate-controlling membrane comprised of ethylene vinyl acetate (EVA) copolymer. The desired membrane permeability is achieved by proper selection of the vinyl acetate (VA) content of the copolymer in addition to the proper annealing conditions. Preferred annealing conditions include for example an annealing temperature of about 45-75°C, most preferred about 52-72ºC, for a period of about 1-72 hours, most preferred about 2-36 hours, and a VA content of 4-18%, most preferred about 5-12%.

In order to facilitate transport of hydrophilic active ingredients through the skin other transdermal embodiments may be applied. Some such methods include iontophoresis, electro-osmosis and electroporation as described by A. K. Banga in the chapter "Electrically Assisted Transdermal Delivery of Drugs" pp. 567-581 and by T. Chen, R. Langer and J. C. Weaver in the chapter "Transdermal Drug Delivery by Skin Electroporation" pp. 597-805 and S. Mitragotri, R. Langer and J. Kost in the chapter enhancement of Transdermal Transport Using Ultrasound in Combination with Other Enhancers" pp. 607-616 and E. R. Scott, J. B. Phipps, J. R. Gyory and R. V. Padmanabhan in the chapter "Electrotransport Systems for Transdermal Delivery: A Practical Implementation of Iontophoresis" pp. 617-659 in the Handbook of Pharmaceutical Controlled Release Technology, Donald L. Wise, ed. Marcel Dekker, Inc., NY 2000. One such embodiment is a transdermal delivery system by Altea Therapeutics which forms micropores in the skin as exemplified by US 6,183,434 B1. An electrotransport and an iontophoretic system are exemplified by US 6,725,090 B1 and US 6,317,529 B1. respectively.

Another embodiment of a transdermal dosage form of the active ingredient is a liquid formulation that is sprayable in droplets as described in US 5,958,379. After being sprayed the composition forms a preparation within a short time, preferably within a time of less than 4 seconds, on the sprayed body surface, particularly on the sprayed skin or mucous membrane, whereby, compared to the original liquid composition, the preparation is formed to have a higher concentration of the active ingredient and which contains the active ingredient in a finely divided way. The composition can be applied by spraying a specific volume to achieve the desired dose.

In order to achieve fast evaporation, such that the liquid composition forms the preparation on the body surface within 4 second, more preferably with a time of less than 2 seconds and most preferable within a time of between 1 second and 0.01 seconds, the composition preferably comprises in addition to the active ingredient such liquids that allow the spraying of droplets with a diameter between 1 micron and 1,000 microns, preferably in droplets having diameters between 10 microns and 100 microns. These additives can be used to affect the surface tension, the zeta potential and viscosity. In one embodiment the liquid composition comprises a viscosity of between 1 mPas and 100 mPas, preferably between 5 mPas and 25 mPas before spraying. The liquid composition is formed such that the active ingredient of the preparation formed on the sprayed body surface is between 25% and 500%, preferably between 50% and 150%, higher that the concentration of the active ingredient of the composition before spraying.

Possible additional ingredients of the liquid formulation include a gel-forming agent. Gel-forming agents may include, but are not limited to, synthetic gel-forming agents such as polymers, particularly polyacrylates, cellulose derivatives, polyvinylalcohols and/or polyvinylacetate. The gel-forming agents can be a natural gel-forming agent, preferably phospholipids or a phospholipids mixture and particularly phospholipids and/or mixture isolated from soybeans, sunflowers and eggs. A particular phospholipid is phosphatidylcholine.

The liquid composition is in the form of a dispersion, a solution and/or a suspension. The composition contains a liquid as a liquid mixture, a pharmaceutical acceptable organic solvent or solvent mixture and/or water. Preferably the liquid composition contains an organic solvent, or as an organic solvent mixture, one or several alcohols which can easily be evaporated. The low molecular weight and easily evaporatable alcohols may include, but are not limited to, ethanol, propanol-1, propanol-2 and/or butanol. The liquid composition may also include propylene glycol alone or in a mixture with the afore mentioned alcohols as organic solvents. A particular embodiment includes a dispersion containing liposomes.

The liquid composition may include other ingredients such as but not limited to preservatives or buffers.

A spraying device is used which preferably operates without a blowing agent and which comprises a pump sprayer, whereby 20 to 300 microliters of the liquid composition per spraying, particularly with a droplet size of between 1 micron and 1,000 microns, preferably with a droplet size of between 10 microns and 100 microns are emitted by means of a suitable spraying nozzle.

Other features and embodiments of the invention will become apparent from the following examples, which are given for illustration of the invention rather than for limiting its intended scope.

### Example 1

As a way of measuring the skin permeation properties of active ingredient, a Franz diffusion cell was provided utilizing a human cadaver skin membrane and a receptor fluid such as 1.0M phosphate buffered saline. The receptor compartment of the Franz diffusion cell was filled with the receptor fluid and the diffusion cell was maintained at 34.5°C. Human cadaver skin was cut out to provide a membrane of 1.767 cm² surface area. The amount of active ingredient that had permeated through the membrane by various times in a 6 to 48 hour period was determined by HPLC analysis of the receptor fluid. Each test was conducted in several replicates. The average calculated skin flux and average amount penetrating per square centimeter after 24 and 48 hours for Formulation I and II are shown in Table 1. These experimental results using the Franz diffusion cell methodology Indicate that transdermal delivery of this compound is feasible based on theoretical skin fluxes for the active ingredient of 0.3 to 5 µg/cm²/hr.

### Formulation Preparation

Formulation I: One gram of varenicline tartrate was dissolved into 5 mL of phosphate buffer (pH 7.0). This formulation was mixed using a vortex mixer and sonicated for 5 minutes. The solution was placed in an amber bottle to limit its exposure to light. The varenicline tartrate did not completely dissolve and the pH of the final solution was 3.41. The resulting saturated solution was used in the in-vitro Franz diffusion cell skin permeation study without filtering to determine the ability of the compound itself to penetrate the skin.

Formulation II: One hundred milligrams of varenicline tartrate were placed in 10 mL of a 50/50 v/v combination of propylene glycol and phosphate buffer solution (pH = 7.0). This formulation was mixed using the vortex mixer and sonicated for 5 minutes. The resulting formulation was a 10 mg/mL non-saturated solution of varenicline tartrate. The actual parent varenicline concentration in this formulation was 5.85 mg/mL. The pH of the final solution was 4.94. This formulation was tested to determine the flux when organic components are also present, in this case propylene glycol was used since it could potentially be used as a solvent and/or a permeation enhancer in either a reservoir style or matrix type device.

### Franz Diffusion Cell Experimental Parameters

| | |
|---|---|
| Apparatus: | Hansen Automated Franz diffusion cell sampling system |
| Cell Volume: | 7.0 mL |
| | |
| Receptor Solution: | Phosphate buffered saline (1.0 M)) |
| Membrane Samples: | Human Cadaver skin, abdomen, male |
| Test Formulations: | Formulation I: Saturated phosphate buffer solution |
| Formulation II: | 1% varenicline tartrate in 50/50 propylene glycol/phosphate buffer solution |
| Dose: | 500 µL over a 1.767 cm² area; occluded |
| Duration: | 48 hours |
| Temperature: | 34.5°C |
| Sample volume:1.0 mL | |
| Rinse volume: | 1.5 mL |
| Sample Analysis: | LC/MS/MS |

**Table 1. Calculated Skin Flux and Amount Penetrating for Formulations I and II**

| | Ave Skin Flux (µg/cm²/hr) | Ave Amt Penetrating @ 24 hrs (µg/cm²) | Ave Amt Penetrating @ 48 hrs (µg/cm²) |
|---|---|---|---|
| Formulation I | 12.58 ± 12.32 | 267 ± 259 | 581 ± 543 |
| Permutation II | 2.41 ± 0.26 | 37.9 ± 16.0 | 49.3 ± 20.0 |

### Example 2 - Matrix type transdermal

The active ingredient is mixed with the aqueous dispersion of NACOR 72-9965 (hydrophobic acrylic copolymer from National Starch) to achieve a 2% (w/w) concentration of active ingredient in the dried film after film casting. The adhesive mixture is cast on a release coated polymer film (Rexam Release Technologies; W. Chicago, IL) and is dried at 60ºC in a convective oven and cut to achieve a 2 mgA dose of the active ingredient. The dried film is laminated to a polyester film laminate (SCOTCHPACK #1012, 3M Pharmaceuticals; St. Paul, MN).

### Example 3 - Matrix type transdermal systems

(1) The base form or salt forms of the active ingredient is dissolved or dispersed in a polyacrylate solution, such as Duro-Tak® 387-2052 adhesive. Appropriate solvents, enhancer and/or filler is added in the adhesive dispersion, and mixed well. Air is removed from the resulting mixture and laminated on a release liner, such as Medirelease® 2228, to form a coating thickness of 0.5 - 2 mm. The adhesive layer is dried at room temperature for 5-10 min and then at 40-80°C for 15 - 30 min to remove all volatile solvents. A backing sheet, such as Mediflex® 1200, is coated on the adhesive side. The resulting patches of a desired size are stored in sealed packages.
(2) The base form or salt forms of the active ingredient is dissolved or dispersed in a polyisobutylene (PIB) based adhesive, such as Duro-Tak® 87-6173. The following procedures are similar to those described in the previous section.
(3) The base form or salt forms of the active ingredient is dissolved or dispersed in a silicone-based adhesive, such as Bio-PSA® 7-4302. The following procedures are similar to those described in the previous section.

### Example 4- Rate-controlling membrane annealed for better stability

The active ingredient is added to a mixture of 95% ethanol and purified water. 2% of hydroxyethyl cellulose gelling agent is added slowly to the active solution with stirring and is mixed until a smooth gel is obtained. A 0.05mm thick contact adhesive layer is formed on a release liner for the system by solution casting an amine resistant silicone medical adhesive (XCF 2992, Dow Coming, Midland, MI) onto the polyester film from a solution in heptane.

An annealed or non-annealed rate-controlling membrane comprised of EVA (9% VA) is pressure laminated to the exposed adhesive to obtain a thickness on the order of 2 - 3 mils. (If annealed, the rate-controlling membrane is kept at 60°C for a period of about 24 hours and then cooled to ambient conditions for about 48 hours before being pressure laminated to the adhesive layer.)

A backing layer comprised of a multilaminate of polyester, polyethylene, aluminum, polyester and EVA (Scotchpak 1220, 3M Co., St. Paul, MN) is used to pouch the active aqueous gel between the backing layer and the release liner/adhesive/rate-controlling membrane using a rotary heat-seal machine. The sealed pouch is die cut in a size to provide the desired amount of active ingredient on the order of 0.5 - 6.0 mg.

### Example 5 - Sprayable liquid

The pharmaceutical composition is manufactured with the following ingredients:
10 g phospholipidic gel-forming agent
16 g ethanol
1 g active ingredient
0.5 g phosphate buffer
distilled water to obtain 100 g of the liquid composition
The 10g of phospholipidic gel-forming agent is hydrated In about 30% by volume of the total amount of water. The active Ingredient and ethanol is added to the hydrated gel-forming agent and mixed in a high pressure homogenizer. The buffer and remaining water is added. Through homogenization an average vesicle diameter of about 100 nm is obtained.

## Claims

1. A transdermal composition comprising varenictine or its pharmaceutically acceptable salt and further comprising a backing sheet having opposing surfaces that are respectively distal and proximal to the skin when applied and a coating on the proximal surface of the backing sheet, wherein the coating comprises (a) an adhesive and (b) varenicline or its pharmaceutically acceptable salt in a water-soluble form in a therapeutically effective total amount and dispensed in a matrix.

2. The transdermal composition of Claim 1, wherein the matrix comprises an adhesive.

3. The transdermal composition of Claim 1, wherein the coating comprises a first reservoir layer which contains the varenicline adjacent to the backing sheet, and a second adhesive layer that Is proximal to the skin when applied, wherein, optionally, a membrane that permits passage of the varenicline is present between the reservoir layer and the adhesive layer.

4. The transdermal composition of Claim 3. wherein the coating further comprises one or more skin permeation enhancers, stabilizers, dyes, pigments, inert fillers, tackiffers, gel forming agents and optional antioxidants.

5. The transdermal composition of Claim 4, wherein the permeation enhancers are chosen from ethanol and other higher alcohols, N-decylmethylsulfoxide (nDMS), polyethylene glycol monolaurate, dilaurate and related esters, glycerol mono-oleate and related mono-, di- and trifunctional glycerides, diethyl toluamide, 1-Dodecylazacycloheptan-2-one, N,N-dimethyl lauramide, N,N-dimethyl lauramine oxide and mixtures thereof.

6. The transdermal composition of Claim 1, wherein the adhesive is selected from an acrylic copolymer, ethylene-vinyl acetate copolymer, and polyisobutylene polymer or copolymer that is a two-phase dispersion of water-insoluble polymer particles in water.

7. The transdermal composition of Claim 1, wherein varenicline is present in an amount which releases from about 0.1 mgA to about 8 mgA per day.

8. The transdermal composition of Claim 7, wherein varenicline is present in an amount which released from about 0.5 mgA to 4 mgA per day.

9. A transdermal composition as defined in any one of claims 1 to 8 for use in reducing nicotine addiction or aiding in the cessation or lessening of tobacco use in a subject, whereinan amount of varenicline that to effective in reducing nicotine addiction or aiding in the cessation or lessening of tobacco use is administered to a subject transdermally.

10. The transdermal composition of Claim 8, wherein the varenicline is present as varenicline tartrate.

11. A method of making a pressure sensitive adhesive matrix patch for transdermal delivery of varenicline by the steps consisting of:
(a) dissolving an effective amount of varenicline in an aqueous dispersion, comprising a water phase, a hydrophobic pressure sensitive adhesive and optionally a permeation enhancer to form a mixture;
(b) film casting the mixture and evaporating the water phase to obtain a hydrophobic pressure sensitive adhesive matrix film having first and second surfaces, the varenicline being fully dissolved therein; and,
(c) laminating a release liner to the first surface of the matrix film and a substantially drug-impermeable backing layer to the second surface, thereby providing a pressure-sensitive adhesive matrix patch for transdermal delivery of varenicline.

12. A sprayable liquid for preparation of a transdermal composition of varenicline, or a pharmaceutically acceptable salt thereof, comprising phospholipidic gel-forming agent, ethanol, varenicline, phosphate buffer, and water.

13. The sprayable liquid of Claim 12, further comprising a phospholipidic gel-forming agent ethanol, phosphate buffer, and water.

14. The transdermal composition of Claim 6, wherein the varenicline is varenicline free base and an antioxidant is present.

## Patentansprüche

1. Transdermale Zusammensetzung, die Vareniclin oder sein pharmazeutisch verträgliches Salz umfasst und die außerdem eine Trägerfolie, die gegenüberliegende Oberflächen hat, welche entsprechend distal und proximal zu der Haut sind, wenn sie appliziert ist, und eine Beschichtung auf der proximalen Oberfläche der Trägerfolie umfasst, wobei die Beschichtung (a) einen Klebstoff und (b) Vareniclin oder sein pharmazeutisch verträgliches Salz in einer wasserlöslichen Form in einer therapeutisch wirksamen Gesamtmenge und dispergiert in einer Matrix umfasst.

2. Transdermale Zusammensetzung gemäß Anspruch 1, wobei die Matrix einen Klebstoff umfasst.

3. Transdermale Zusammensetzung gemäß Anspruch 1, wobei die Beschichtung eine erste Reservoirschicht, die das Vareniclin enthält, angrenzend an die Trägerfolie und eine zweite Klebstoffschicht, die proximal zu der Haut ist, wenn sie appliziert ist, umfasst, wobei gegebenenfalls eine Membran, welche eine Passage des Vareniclins erlaubt, zwischen der Reservoirschicht und der Klebstoffschicht vorhanden ist.

4. Transdermale Zusammensetzung gemäß Anspruch 3, wobei die Beschichtung außerdem ein oder mehr Verstärkungsmittel für die Hautpermeation, Stabilisatoren, Farbstoffe, Pigmente, inerte Füllstoffe, Klebrigmacher, Gel bildende Mittel und optionale Antioxidantien umfasst.

5. Transdermale Zusammensetzung gemäß Anspruch 4, wobei die Verstärkungsmittel für die Permeation ausgewählt sind aus Ethanol und anderen höheren Alkoholen, N-Decylmethylsulfoxid (NDMS), Polyethylenglycolmonolaurat, -dilaurat und verwandten Estern, Glycerinmonooleat und verwandten mono-, di- und trifunktionellen Glyceriden, Diethyltoluamid, 1-Dodecylazacycloheptan-2-on, N,N-Dimethyllauramid, N,N-Dimethyllauraminoxid und Gemischen davon.

6. Transdermale Zusammensetzung gemäß Anspruch 1, wobei der Klebstoff aus einem Acrylcopolymer, Ethylen-vinylacetar-Copolymer und Polyisobutylenpolymer oder -copolymer ausgewählt ist, der eine Zwei-Phasen-Dispersion von wasserunlöslichen Polymerpartikeln in Wasser ist.

7. Transdermale Zusammensetzung gemäß Anspruch 1, wobei Vareniclin in einer Menge vorliegt, die etwa 0,1 mgA bis etwa 6 mgA pro Tag freisetzt.

8. Transdermale Zusammensetzung gemäß Anspruch 7, wobei Vareniclin in einer Menge vorliegt, die etwa 0,5 mgA bis 4 mgA pro Tag freisetzt.

9. Transdermale Zusammensetzung, wie sie in einem der Ansprüche 1 bis 8 definiert ist, zur Verwendung bei der Verringerung der Nikotinabhängigkeit oder bei der Unterstützung des Beendens oder Verringerns der Tabakverwendung bei einem Subjekt, wobei eine Menge an Vareniclin, die bei der Verringerung der Nikotinabhängigkeit oder bei der Unterstützung des Beendens oder Verringerns der Tabakverwendung wirksam ist, transdermal an ein Subjekt verabreicht wird.

10. Transdermale Zusammensetzung wie in Anspruch 9 definiert, wobei das Vareniclin als Vareniclin-Tartrat vorliegt.

11. Verfahren zur Herstellung eines druckempfindlichen Klebstoff-Matrix-Pflasters zur transdermalen Abgabe von Vareniclin durch die Schritte, bestehend aus:
(a) Lösen einer wirksamen Menge an Vareniclin in einer wässrigen Dispersion, die eine Wasserphase, einen hydrophoben druckempfindlichen Klebstoff und gegebenenfalls einen Permeationsverstärker umfasst, unter Bildung eines Gemisches;
(b) Filmgießen des Gemisches und Verdampfen der Wasserphase unter Erhalt eines Films aus hydrophober druckempfindlicher Klebstoffmatrix, der erste und zweite Oberflächen hat, wobei das Vareniclin vollständig darin gelöst ist, und
(c) Laminieren einer Decklage auf die erste Oberfläche des Matrixfilms und einer im Wesentlichen arzneimittelundurchlässigen Rückseitenschicht auf die zweite Oberfläche, wodurch ein druckempfindliches Klebstoff-Matrix-Pflaster zur transdermalen Abgabe von Vareniclin bereitgestellt wird.

12. Sprühfähige Flüssigkeit zur Herstellung einer transdermalen Zusammensetzung von Vareniclin oder einem pharmazeutisch verträglichen Salz davon.

13. Sprühfähige Flüssigkeit gemäß Anspruch 12, die außerdem ein Phospholipid-Gelbildungsmittel, Ethanol, Phosphatpuffer und Wasser umfasst.

14. Transdermale Zusammensetzung gemäß Anspruch 6, wobei das Vareniclin die freie Base von Vareniclin ist und ein Antioxidans vorliegt.

## Revendications

1. Composition transdermique comprenant de la varénicline ou son sel pharmaceutiquement acceptable et comprenant, en outre, une feuille-dossier ayant des surfaces opposées qui, en service, sont respectivement distale et proximale par rapport à la peau et une enduction sur la surface proximale de la feuille-dossier, l'enduction comprenant (a) un adhésif et (b) de la varénicline ou son sel pharmaceutiquement acceptable, sous une forme soluble dans l'eau, en une quantité totale thérapeutiquement efficace et dispersée dans une matrice.

2. Composition transdermique selon la revendication 1, dans laquelle la matrice comprend un adhésif.

3. Composition transdermique selon la revendication 1, dans laquelle l'enduction comprend une première couche-réservoir qui contient la varénicline adjacente à la feuille-dossier, et une seconde couche adhésive qui, en service, est proximale à la peau, une membrane permettant le passage de la varénicline étant facultativement présente entre la couche réservoir et la couche adhésive.

4. Composition transdermique selon la revendication 3, dans laquelle l'enduction comprend en outre un ou plusieurs améliorateurs de la perméation de la peau, stabilisants, colorants, pigments, charges inertes, agents de pégosité, agents gélifiants et antioxydants facultatifs.

5. Composition transdermique selon la revendication 4, dans laquelle les améliorateurs de perméation sont choisis parmi l'éthanol et d'autres alcools supérieurs, le N-décylméthylsulfoxyde (nDMS), le monolaurate, dilaurate et autres esters apparentés de polyéthylène glycol, le mono-oléate de glycérol et les glycérides mono-, di- et trifonctionnels apparentés, le diéthyltoluamide, la 1-dodécylazacycloheptan-2-one, le N,N-diméthyl-lauramide, l'oxyde de N,N-diméthyl-lauramine et leurs mélanges.

6. Composition transdermique selon la revendication 1, dans laquelle l'adhésif est sélectionné parmi un copolymère acrylique, un copolymère éthylène-acétate de vinyle et un polymère ou copolymère polyisobutylène qui est une dispersion biphasique de particules de polymère hydro-insolubles dans de l'eau.

7. Composition transdermique selon la revendication 1, dans laquelle de la varénicline est présente en une quantité qui libère d'environ 0,1 mgA à environ 6 mgA par jour.

8. Composition transdermique selon la revendication 7, dans laquelle de la varénicline est présente en une quantité qui libère d'environ 0,5 mgA à 4 mgA par jour.

9. Composition transdermique telle que définie dans l'une quelconque des revendications 1 à 8, pour une utilisation dans la réduction de l'addiction à la nicotine ou l'aide à l'arrêt ou à la diminution du tabagisme chez un sujet, une quantité de varénicline efficace pour réduire l'addiction à la nicotine ou aider à l'arrêt ou à la diminution du tabagisme étant administrée à un sujet par voie transdermique.

10. Composition transdermique telle que définie dans la revendication 9, la varénicline étant présente sous forme de tartrate de varénicline.

11. Procédé de préparation d'un timbre à matrice adhésive sensible à la pression pour l'administration transdermique de varénicline selon les étapes suivantes :
(a) dissolution d'une quantité efficace de varénicline dans une dispersion aqueuse, comprenant une phase aqueuse, un adhésif hydrophobe sensible à la pression et, facultativement, un améliorateur de perméation, pour former un mélange ;
(b) coulage du mélange sous la forme d'un film et évaporation de la phase aqueuse pour obtenir un film de matrice adhésive, hydrophobe, sensible à la pression ayant une première et une seconde surfaces, la varénicline y étant totalement dissoute ; et
(c) lamination d'une doublure anti-adhérente sur la première surface du film de matrice et d'une couche-dossier sensiblement imperméable aux médicaments sur la seconde surface, offrant ainsi un timbre de matrice adhésive sensible à la pression pour administration transdermique de varénicline.

12. Liquide pulvérisable pour la préparation d'une composition transdermique de varénicline, ou d'un sel pharmaceutiquement acceptable de celle-ci.

13. Liquide pulvérisable selon la revendication 12, comprenant en outre un agent gélifiant phospholipidique, de l'éthanol, du tampon phosphate et de l'eau.

14. Composition transdermique selon la revendication 6, dans laquelle la varénicline est sous forme de base libre et un antioxydant est présent.
